# EUROPÄISCHE PATENTSCHRIFT

(11) **EP 3 613 446 B1**
(45) Veröffentlichungstag und Bekanntmachung des Hinweises auf die Patenterteilung: **17.05.2023**
(21) Anmeldenummer: 19185358.9
(22) Anmeldetag: 10.07.2019
(51) Int. Cl.: A61L 27/30, A61L 27/32, A61L 27/54, A61L 29/10, A61L 29/16, A61L 31/08, A61L 31/16

(54) **IMPLANTIERBARES MEDIZINPRODUKT MIT DAUERHAFT NEGATIV GELADENER OBERFLÄCHE**
IMPLANTABLE MEDICAL DEVICE WITH PERMANENTLY NEGATIVELY CHARGED SURFACE
DISPOSITIF MÉDICAL POUVANT ÊTRE IMPLANTÉ POURVU DE SURFACE CHARGÉE NÉGATIVEMENT DE MANIÈRE PERMANENTE

(30) Priorität: 23.08.2018 DE 102018214299
(43) Veröffentlichungstag der Anmeldung: 26.02.2020
(73) Patentinhaber: UroNova GmbH medizinische Implantate, 91083 Baiersdorf (DE)
(72) Erfinder: Hildebrandt, Peter, 91074 Herzogenaurach (DE)
(74) Vertreter: Rau, Schneck & Hübner Patentanwälte Rechtsanwälte PartGmbB

(56) Entgegenhaltungen:
- EP-A2- 0 761 244
- WO-A2-2005/032417
- DE-A1- 2 622 394
- US-A- 4 882 148
- US-A- 5 939 208
- BALAZS D J ET AL: "Inhibition of bacterial adhesion on PVC endotracheal tubes by RF-oxygen glow discharge, sodium hydroxide and silver nitrate treatments", BIOMATERIALS, ELSEVIER SCIENCE PUBLISHERS BV., BARKING, GB, Bd. 25, Nr. 11, 1. Mai 2004 (2004-05-01), Seiten 2139-2151, XP004485132, ISSN: 0142-9612, DOI: 10.1016/J.BIOMATERIALS.2003.08.053
- EMERSON ET AL: "Microscale Correlation between Surface Chemistry, Texture, and the Adhesive Strength of Staphylococcus e pidermidis", LANGMUIR, Bd. 22, Nr. 26, 1. Dezember 2006 (2006-12-01), Seiten 11311-11321, XP055635769, US ISSN: 0743-7463, DOI: 10.1021/la061984u
- CHENG ET AL: "Inhibition of bacterial adhesion and biofilm formation on zwitterionic surfaces", BIOMATERIALS, ELSEVIER SCIENCE PUBLISHERS BV., BARKING, GB, Bd. 28, Nr. 29, 10. August 2007 (2007-08-10), Seiten 4192-4199, XP022192627, ISSN: 0142-9612, DOI: 10.1016/J.BIOMATERIALS.2007.05.041

## Beschreibung

Die vorliegende Patentanmeldung nimmt die Priorität der deutschen Patentanmeldung DE 10 2018 214 299.8 in Anspruch.

Die Erfindung betrifft ein ganz oder teilweise implantierbares Medizinprodukt mit einer negativ geladenen Oberfläche zur Abstoßung von Bakterien, gekennzeichnet durch einen oberflächlich angebundenen Stoff mit einem dauerhaften negativen Ladungsüberschuss, welcher Stoff gegenüber Zellen des menschlichen Körpers und sich in diesem befindlichen Bakterien inert ist sowie keine pharmakologische oder anderweitig schädliche Wirkung zeigt, wobei eine oder mehrere verschiedene Phosphon- oder Sulfonsäuren als negative Ladungen tragender Stoff kovalent über eine Peptidbindung an die Oberfläche angebunden und deprotoniert sowie die so entstandene negative Ladung durch Ausbildung einer Mesomerie stabilisiert sind.

Der Hintergrund der Erfindung ist wie folgt zu schildern. Es sind die Besiedlung von implantierbaren Medizinprodukten mit Bakterien, Biofilm und Inkrustationen und die dadurch entstehenden Probleme bei der Behandlung von Patienten seit vielen Jahren Gegenstand der Forschung. Als Beispiele für solche implantierbaren Medizinprodukte seien Stents, Katheter, Netzimplantate, Brustimplantate und Herzschrittmacher genannt, wobei es eine große Anzahl an weiteren Beispielen gibt. Durch die bakterielle Besiedlung kann es zu Biofilmbildung und Infektionen kommen, weiterhin gibt es dadurch ausgelöste Effekte, wie den Verschluss von röhrenförmigen Implantaten durch Ablagerungen und Inkrustation. Die Anbindung von pharmakologischen Wirkstoffen an der Oberfläche von implantierbaren Medizinprodukten hat sich in diesem Zusammenhang teilweise als erfolgreich erwiesen. So sind dem Stand der Technik zahlreiche Druckschriften zu entnehmen, die beispielsweise eine kovalente Anbindung von antibiotischen Wirkstoffen an die Oberfläche vorschlagen wie beispielsweise in US 2011/135703 A1 und AU 2013245551 B2.

Im Patent EP 0 890 367 B1 wird die kovalente Anbindung von Glykosaminoglykanen an ein urologisches Implantat vorgeschlagen. Wie spätere Forschungsarbeiten gezeigt haben, hat sich dieses Konzept durchaus als erfolgreich erwiesen. Als entscheidender Faktor für die Wirksamkeit wird die negative Ladung der Glykosaminoglykane angesehen (Int J Antimicrob Agents. 2004, 23: Bacterial biofilm formation on urologic devices and heparin coating as preventive strategy. Tenke P. et al.). Da Bakterien gleichermaßen über einen negativen Ladungsüberschuss verfügen, kommt es zu einer elektrostatischen Abstoßung von der mit Glykosaminoglykanen beschichteten Oberfläche. Heparin ist dabei das Glykosaminoglykan mit der höchsten negativen Ladung. Die negative Ladung der Bakterien kommt im Falle von Gram negativen Bakterien von den stark negativ geladenen Lipopolysacchariden in der Zellwand, im Falle von Gram positiven Bakterien von der negativ geladenen Phosphatgruppe der Phosphodiesterbindungen zwischen den Monomeren der Teichonsäuren in der Zellwand.

Zum allgemeinen Stand der Technik ist zudem auf die Fachveröffentlichung von Shetal AnilKumar et al., "Fabrication of Antibacterial Coatings: Prevention of Implant Associated Infections in Patients Indwelling Urinary Catheters", Journal of Pharmacy and Biological Sciences, Vol. 10, Issue 6 ver. III (Nov. - Dec. 2015), S. 82 - 89 zu verweisen.

Durch die Anbindung von Substanzen mit pharmakologischer Wirkung ergeben sich jedoch auch unterschiedliche Probleme. Als pharmakologische Wirkung wird eine Wechselwirkung zwischen der Substanz und einem zellulären Bestandteil des Körpers des Patienten oder einem im Körper des Patienten vorhandenen zellulären Bestandteil verstanden. Letztere sind beispielsweise Bakterien oder Pilze im Körper des Patienten, die beispielsweise mit bioziden oder antibiotischen Wirkstoffen wechselwirken. Neben der gewollten Erzielung einer negativ geladenen Oberfläche zur Abstoßung von Bakterien kann durch die Anbindung von pharmakologischen Wirkstoffen zusätzlich eine ungewollte Wirkung erzielt werden. Bei der Verwendung der Glykosaminoglykane Heparin und Heparansulfat kann das die blutgerinnungshemmende Wirkung sein, die beispielsweise bei perkutanen Kathetern wie Nephrostomiekathetern oder suprapubischen Blasenkathetern zu einer erhöhten Blutungsneigung führen kann, was der Verwendung so ausgestatteter Katheter entgegensteht. Beim Glykosaminoglykan Hyaluronsäure wurde eine verstärkende Wirkung auf die Zellproliferation festgestellt und es findet in Präparaten zur Erhaltung der Gelenkfunktion Verwendung. Die Beeinflussung der Zellproliferation kann beispielsweise bei Gefäßwandstützen zu unerwünschten Effekten führen, wie etwa einem Zuwachsen derselben durch proliferierendes Gewebe.

Ein anderes Problem ist die Klassifizierung der Medizinprodukte. In manchen Ländem werden Medizinprodukte, an deren Oberfläche pharmakologische Wirkstoffe angebunden sind - auch wenn diese Anbindung langzeitstabil und ohne die Gefahr einer Wirkstofffreigabe erfolgt - als Kombinationsprodukte klassifiziert. Dies hat zur Folge, dass Richtlinien für Pharmaprodukte befolgt werden müssen und sich der Zulassungs- und Vertriebsaufwand signifikant erhöht.

Die WO 2005/032417 A2 offenbart eine antimikrobielle Beschichtung für orthopädische Implantate mit Hyualuronsäure, die sich auf die Beeinflussung der Zellproliferation negativ auswirkt.

Die DE 26 22 394 A1 beschreibt einen Implantationskatheter, bei dem die äußere Oberfläche durch die Verwendung eines Acrylsäure-Ethylen-Copolymers zum Einführen von Carboxylgruppen beschichtet wird. Dabei ist das Grundmaterial des Katheters dahingehend eingeschränkt, dass es nicht dehnbar sein darf.

Die US 5 939 208 A schlägt für medizinische Produkte die Verwendung eines Polymers mit einer negativ geladenen Oberfläche vor, indem Hydrogele chemisch umgewandelt werden oder eine Hydrogel-Beschichtung vorgenommen wird, die dann chemisch umgewandelt wird. Aufgrund der Dicken von Hydrogel-Beschichtungen sind diese für Katheter mit filigranen Formmerkmalen, wie dünnen Lumen, nicht geeignet.

Die Fachveröffentlichung D.J. Balazs et al. "Inhibition ofbacterial adhesion..." Biomaterials 25 (2004) 2139-2151 beschreibt ein Verfahren zur Immobilisierung von Silberionen zur Herstellung einer PVC-Oberfläche mit Eigenschaften, die eine bakterielle Kolonisierung hemmen. Diese Wirkung wird durch Silberionen erzielt, welche biozid sind.

Die in der Fachveröffentlichung Emerson et al. "Microscale Correlation between Surface Chemistry..." Langmuir 22 (2006) 11311 - 11321 beschriebene Anbindung von Isophthalaten auf Gold erfolgt über die Ausbildung einer selbstorganisierenden Monoschicht (SAM). Dabei findet keine langzeitstabile Anbindung der Moleküle an das Substrat statt.

Die US 4 882 148 A offenbart Methoden, die Rissbildung bei implantierbaren Polymeren zu hemmen und antithrombogene Eigenschaften herzustellen. Die Erfindung zielt grundsätzlich nicht auf Bakterien abstoßende Eigenschaften ab. Bei dem in diesem Stand der Technik eingesetzten Verfahren werden rauchende Schwefelsäure, konzentrierte Schwefelsäure oder Schwefeltrioxid-Dämpfe angewandt, um Sulfonatgruppen an der Oberfläche einzuführen. Diese hochgefährlichen, ätzenden und teils explosiven Stoffe erhöhen den Produktionsaufwand und dessen Kosten immens und sind von der Rentabilität her nicht akzeptabel.

Vor diesem Hintergrund stellt sich der Stand der Technik als verbesserungswürdig dar.

Zur Lösung der geschilderten Probleme des Standes der Technik schlägt die Erfindung laut Kennzeichnungsteil des Patentanspruches 1 als grundsätzliches Konzept vor, einen Stoff mit einem dauerhaften negativen Ladungsüberschuss oberflächlich anzubinden, welcher Stoff gegenüber Zellen des menschlichen Körpers und sich in diesem befindlichen Bakterien inert verhält. Damit wird eine Modifikation der Oberfläche eines implantierbaren Medizinproduktes zur Herstellung einer dauerhaften negativen Ladung ohne die Verwendung von Stoffen realisiert, die eine pharmakologische oder anderweitig schädliche Wirkung haben. Damit werden die eingangs geschilderten Probleme ohne Einbußen an Wirksamkeit rundum vermieden. Um eine langzeitstabile Anbindung an die Oberfläche des Medizinproduktes zu erreichen, ist dabei eine kovalente Bindung in Form einer Peptidbindung vorgesehen.

Für den oberflächlich angebundenen Stoff sind verschiedene Realisierungsmöglichkeiten gegeben. Ganz grundsätzlich ist dabei darauf zu achten, dass dieser Stoff seinen negativen Ladungsüberschuss an der Oberfläche dauerhaft behält und seine negative Ladung nicht durch chemische Reaktionen im Implantationsbereich neutralisiert wird. Solche Reaktionen können beispielsweise im Kontakt mit Gewebe und Körperflüssigkeiten wie Blut, Urin oder Wundsekreten stattfinden.

Dabei werden erfindungsgemäß eine oder mehrere verschiedene Phosphon- oder Sulfonsäuren als negative Ladungen tragender Stoff kovalent über eine Peptidbindung an die Oberfläche angebunden und deprotoniert sowie die so entstandene negative Ladung durch Ausbildung einer Mesomerie stabilisiert.

Erfindungsgemäß sind Sulfonsäuren oder Phosphonsäuren vorgesehen, welche deprotoniert werden können und durch Ausbildung einer Mesomerie ihre negative Ladung stabilisieren.

Ein geeigneter Vertreter der Sulfonsäuren ist gemäß Anspruch 2 beispielsweise 6-Aminonaphthalin-2-sulfonsäure, die über eine Aminogruppe verfügt und sich damit über eine Peptidbindung kovalent anbinden lässt.

Ein geeigneter Vertreter der Phosphonsäuren ist gemäß Anspruch 3 beispielsweise (3-Aminopropyl)phosphonsäure, die ebenfalls über eine Aminogruppe verfügt und sich damit über eine Peptidbindung kovalent anbinden lässt.

Eine weitere bevorzugte Ausführungsform gemäß Anspruch 4 sieht eine Deprotonierung des Stoffes mithilfe von verdünnter Natronlauge vor.

Es folgt eine nähere Erläuterung mit der Erfindung anhand verschiedener

### Ausführungsbeispiele:

**Beispiel I** (nicht erfindungsgemäß) - Kovalentes Beschichtungsverfahren mit Tricarballylsäure auf einem medizinischen Implantat mit Silikonoberfläche
   1) Dazu wird die Silikonoberfläche zur Vorbereitung der Anbindung von Tricarballylsäure für 12 h in eine wässrige 3-Aminopropyltriethoxysilan-Lösung eingelegt und anschließend in deionisiertem Wasser gespült.
   2) Die so vorbereitete Silikonoberfläche wird zur Anbindung der Tricarballylsäure über eine Peptidbindung für 12 h in eine wässrige Lösung aus Tricarballylsäure und N-(3-Dimethylaminopropyl)-N'-ethylcarbodiimid eingelegt und anschließend in deionisiertem Wasser gespült.
   3) Zur Deprotonierung der freien Carboxygruppen der Tricarballylsäure wird die so vorbereitete Silikonoberfläche nun für 6 h in eine 10%ige Natronlauge eingelegt und anschließend in deionisiertem Wasser gespült.
**Beispiel II** (erfindungsgemäß) - Kovalentes Beschichtungsverfahren mit 6-Aminonaphthalin-2-sulfonsäure auf einer Polyurethanoberfläche
   1) Dazu wird die Polyurethanoberfläche zur Vorbereitung der Anbindung von Taurin für 12 h in eine Hexamethylendiisocyanat-Lösung in Ether eingelegt und anschließend in deionisiertem Wasser gespült.
   2) Die so vorbereitete Polyurethanoberfläche wird dann zur Hydrolyse für 12 h in eine wässrige Lösung aus Natriumhydrogencarbonat eingelegt und anschließend in deionisiertem Wasser gespült.
   3) Die so vorbereitete Polyurethanoberfläche wird dann für 12 h in eine wässrige Lösung aus Malonsäure und N-(3-Dimethylaminopropyl)-N'-ethylcarbodiimid eingelegt und anschließend in deionisiertem Wasser gespült.
   4) Die so vorbereitete Polyurethanoberfläche wird zur Vorbereitung der Anbindung von Taurin für 1 h in eine wässrige Lösung aus N-(3-Dimethylaminopropyl)-N'-ethylcarbodiimid eingelegt.
   5) Die so vorbereitete Polyurethanoberfläche wird zur Anbindung von 6-Aminonaphthalin-2-sulfonsäure über eine Peptidbindung für 12 h in eine wässrige Lösung aus 6-Aminonaphthalin-2-sulfonsäure eingelegt und anschließend in deionisiertem Wasser gespült.
   6) Zur Deprotonierung der freien Carboxygruppe der 6-Aminonaphthalin-2-sulfonsäure wird die so vorbereitete Polyurethanoberfläche nun für 6 h in eine 10%ige Natronlauge eingelegt und anschließend in deionisiertem Wasser gespült.
**Beispiel III** (erfindungsgemäß) - Kovalentes Beschichtungsverfahren mit (3-Aminopropyl)phosphonsäure auf einer Polyurethanoberfläche
   1) Dazu wird die Polyurethanoberfläche zur Vorbereitung der Anbindung von (3-Aminopropyl)phosphonsäure für 12 h in eine Hexamethylendiisocyanat-Lösung in Ether eingelegt und anschließend in deionisiertem Wasser gespült.
   2) Die so vorbereitete Polyurethanoberfläche wird dann zur Hydrolyse für 12 h in eine wässrige Lösung aus Natriumhydrogencarbonat eingelegt und anschließend in deionisiertem Wasser gespült.
   3) Die so vorbereitete Polyurethanoberfläche wird dann für 12 h in eine wässrige Lösung aus Malonsäure und N-(3-Dimethylaminopropyl)-N'-ethylcarbodiimid eingelegt und anschließend in deionisiertem Wasser gespült.
   4) Die so vorbereitete Polyurethanoberfläche wird zur Vorbereitung der Anbindung von (3-Aminopropyl)phosphonsäure für 1 h in eine wässrige Lösung aus N-(3-Dimethylaminopropyl)-N'-ethylcarbodiimid eingelegt.
   5) Die so vorbereitete Polyurethanoberfläche wird zur Anbindung von (3-Aminopropyl)phosphonsäure über eine Peptidbindung für 12 h in eine wässrige Lösung aus (3-Aminopropyl)phosphonsäure eingelegt und anschließend in deionisiertem Wasser gespült.
   6) Zur Deprotonierung der freien Carboxygruppe der (3-Aminopropyl)phosphonsäure wird die so vorbereitete Polyurethanoberfläche nun für 6 h in eine 10%ige Natronlauge eingelegt und anschließend in deionisiertem Wasser gespült.

**Nachweistest zu Beispiel I** (nicht erfindungsgemäß) - Langzeitstabile Anbindung von Tricarballylsäure mit negativ geladenen Carboxylatgruppen an Silikonoberfläche Der Nachweis wird mithilfe von Toluidin Blau durchgeführt. Letzteres ist ein positiv gefärbter Farbstoff, der an die negativ geladenen Carboxylatgruppen ionisch anbindet. Zum Nachweis der erfolgreichen langzeitstabilen Anbindung von Tricarballylsäure mit negativ geladenen Carboxylatgruppen nach oben beschriebenem Verfahren wurden folgende Schritte durchgeführt:
A) Spülen der aus Beispiel I gewonnenen Proben für 7 Tage in physiologische Kochsalzlösung zum Entfernen adsorbierter, nicht kovalent angebundener Tricarballylsäure unter dauernder Umströmung mit Hilfe eines Magnetrührers.
B) Vorbereitung einer 0,1%igen wässrigen Lösung von Toluidin Blau und photospektrometrische Messung der Transmission bei 640 nm.
C) Einlegen einer wie unter A) beschrieben gespülten Probe in die unter B) beschriebene Lösung für 15 Minuten bei 60°C.
D) Photospektrometrische Messung der Transmission bei 640 nm und Vergleich mit dem unter B) gemessenen Transmissionswert.

Das Toluidin Blau, das aus der Lösung an die Oberfläche der Proben ionisch anbindet, trägt nicht mehr zur Blaufärbung der Lösung bei. Da nach 15 Minuten von einem nahezu vollständigen Ladungsausgleich an der Oberfläche auszugehen ist, kann aus der Erhöhung der Transmission auf die Menge von negativ geladenen Carboxylatgruppen geschlossen werden. In Experimenten konnte so eine Flächenbeladung von etwa 700 pMol/cm² Tricarballylsäure auf wie oben beschrieben beschichteten Proben gemessen werden.

Die mikrobiologische Untersuchung von so ausgestatteten Proben konnte nach 24 stündiger Lagerung bei 37°C in Suspensionen mit Escherischia Coli Bakterien in physiologischer Kochsalzlösung mit einer Konzentration von 1,5 Millionen koloniebildenden Einheiten pro ml eine mehr als 95% geringere Anlagerung von Bakterien an der Oberfläche nachweisen. Weiterhin wurden mit 6-Aminonaphthalin-2-sulfonsäure beschichtete Silikonkatheter in gepoolten menschlichen Urin künstlich mit Proteus mirabilis Bakterien infiziert, in einem Modell der Menschlichen Harnblase nach Stickler (Stickler DJ, Morris NS, Winters C. Simple physical model to study formation and physiology of biofilms on urethral catheters. Methods Enzymol. 1999; 310:494-501) getestet. Während unbeschichtete Silikonkatheter bereits nach 40 Stunden durch kristalline und organische Ablagerungen verschlossen waren, behielten die beschichteten Silikonkatheter auch nach 96 Stunden noch ihre Durchgängigkeit.

## Patentansprüche

1. Ganz oder teilweise implantierbares Medizinprodukt mit einer negativ geladenen Oberfläche zur Abstoßung von Bakterien, **gekennzeichnet durch** einen oberflächlich angebundenen Stoff mit einem dauerhaften negativen Ladungsüberschuss, welcher Stoff gegenüber Zellen des menschlichen Körpers und sich in diesem befindlichen Bakterien inert ist sowie keine pharmakologische oder anderweitig schädliche Wirkung zeigt, wobei eine oder mehrere verschiedene Phosphon- oder Sulfonsäuren als negative Ladungen tragender Stoff kovalent über eine Peptidbindung an die Oberfläche angebunden und deprotoniert sowie die so entstandene negative Ladung durch Ausbildung einer Mesomerie stabilisiert sind.

2. Medizinprodukt nach Anspruch 1, **dadurch gekennzeichnet, dass** es sich bei der verwendeten Sulfonsäure um 6-Aminonaphthalin-2-sulfonsäure handelt.

3. Medizinprodukt nach Anspruch 1, **dadurch gekennzeichnet, dass** es sich bei der verwendeten Phosphonsäure um (3-Aminopropyl)phosphonsäure handelt.

4. Medizinprodukt nach einem der vorgenannten Ansprüche, **dadurch gekennzeichnet, dass** der Stoff mit Hilfe von verdünnter Natronlauge deprotoniert ist.

## Claims

1. Medical product, which is either entirely or partially implantable, with a negatively charged surface for repulsing bacteria, **characterized by** a superficially bonded substance with a permanent negative excess charge, which substance is inert against cells of the human body and the bacteria contained therein and does not show any pharmacological or otherwise harmful effect, wherein one or more different phosphonic or sulfonic acids, as substance carrying negative charges, are covalently bonded to the surface via a peptide bond and deprotonated and the resulting negative charge is stabilized by the establishment of a mesomerism.

2. Medical device according to claim 1, **characterized in that** the sulfonic acid used is 6-aminonaphthalene-2-sulfonic acid.

3. Medical device according to claim 1, **characterized in that** the phosphonic acid used is (3-aminopropyl)phosphonic acid.

4. Medical device according to one of the preceding claims, **characterized in that** the substance is deprotonated with the help of diluted sodium hydroxide.

## Revendications

1. Produit médical pouvant être implanté entièrement ou partiellement pourvu d'une surface chargée négativement pour repousser les bactéries, **caractérisé par** une substance liée en surface avec un excédent de charge négative durable, laquelle substance est inerte vis-à-vis des cellules du corps humain et des bactéries qui s'y trouvent et ne présente pas d'effet pharmacologique ou autrement nocif, un ou plusieurs acides phosphoniques ou sulfoniques différents étant liés de manière covalente à la surface par une liaison peptidique en tant que substance portant des charges négatives et déprotonés, et la charge négative ainsi créée étant stabilisée par la formation d'une mésomérie.

2. Produit médical selon la revendication 1, **caractérisé en ce que** l'acide sulfonique utilisé est l'acide 6-aminonaphtalène-2-sulfonique.

3. Produit médical selon la revendication 1, **caractérisé en ce que** l'acide phosphonique utilisé est l'acide (3-aminopropyl)phosphonique.

4. Produit médical selon l'une des revendications précédentes, **caractérisé en ce que** la substance est déprotonée à l'aide de soude caustique diluée.
